# EUROPÄISCHE PATENTSCHRIFT

(11) **EP 4 431 143 B1**
(45) Veröffentlichungstag und Bekanntmachung des Hinweises auf die Patenterteilung: **02.09.2026**
(21) Anmeldenummer: 24192747.4
(22) Anmeldetag: 23.10.2019
(51) Int. Cl.: B05B 5/16, B05B 12/12, B05B 12/00, A61M 35/00

(54) **VERFAHREN ZUM AUFTRAGEN EINER KOSMETISCHEN SUBSTANZ AUF DIE HAUT EINER PERSON**
METHOD FOR APPLYING A COSMETIC SUBSTANCE TO THE SKIN OF A PERSON
PROCÉDÉ D'APPLICATION D'UNE SUBSTANCE COSMÉTIQUE SUR LA PEAU D'UNE PERSONNE

(30) Priorität: 24.10.2018 DE 102018126568
(43) Veröffentlichungstag der Anmeldung: 18.09.2024
(62) Dokumentnummer(n) der früheren Anmeldung(en) nach Art. 76 EPÜ: 19794964.7 / 3 870 373
(73) Patentinhaber: IONIQ Skincare GmbH & Co. KG, 88677 Markdorf (DE)
(72) Erfinder: Langen, Valentin, 88662 Überlingen (DE)
(74) Vertreter: Patentanwälte Olbricht Buchhold Keulertz

(56) Entgegenhaltungen:
- EP-A1- 3 375 323
- WO-A1-2018/193068
- WO-A1-2019/103974
- US-A1- 2016 022 011

## Beschreibung

Die Erfindung betrifft ein nicht-therapeutisches Verfahren zum Auftragen einer kosmetischen Substanz auf die Haut einer Person gemäß Anspruch 1 sowie ein Gerät zum Auftragen einer kosmetischen Substanz auf die Haut einer Person gemäß Anspruch 18.

Kosmetische Substanzen, wie z.B. Hautpflegeprodukte, Lotionen, Sonnenschutzmittel, Selbstbräuner u.dgl., werden gewöhnlich von Hand auf die Haut einer Person aufgetragen. Ein solches Verfahren ist - insbesondere, wenn größere Hautbereiche zu behandeln sind - zeitaufwändig und ein nicht unerheblicher Teil der Substanz geht in den Händen verloren. Um den Kontakt mit der kosmetischen Substanz durch den Auftragenden zu minimieren und um eine verbesserte Verteilung der Substanz zu erlangen, werden Pumpsprays oder unter Druck stehende Aerosolbehälter angeboten, welche die kosmetische Substanz enthalten und mit Hilfe eines Treibgases zerstäuben und versprühen. Ein Nachteil hierbei ist jedoch, dass ein Großteil der Substanz nicht auf die Haut gelangt, sondern in die nähere Umgebung, was unerwünscht ist.

Für einen gezielteren Auftrag einer kosmetischen Substanz auf die Haut einer Person wurden ferner Sprühgeräte konzipiert, die eine elektrostatische Zerstäubung vorsehen. Hierbei werden die Partikel oder Tröpfchen der zu versprühenden Substanz mittels einer Hochspannungselektrode elektrisch geladenen und aufgrund elektrischer Feldkräfte auf die Haut der zu behandelnden Person gerichtet, wobei die Haut bezogen auf das Sprühgerät geerdet ist.

Elektrostatische Zerstäubung umfasst im Sinne der vorliegenden Erfindung alle Zerstäubungs- oder Vernebelungsvorgänge, welche Substanzen mit Effekten der Einwirkung einer Hochspannung zerstäuben oder vernebeln. Insbesondere auch elektrohydrodynamische Effekte sowie elektrokinetische Effekte sind von dem Begriff dieser Art von Zerstäubung umfasst. Im Sinne der Erfindung kann unter einer elektrostatischen Zerstäubung auch eine elektrohydrodynamische Zerstäubung aufgefasst werden.

Derartige elektrostatische Sprühgeräte haben - wie beispielsweise in WO 2002 055 211 A1 offenbart - einen Behälter für das zu versprühende Produkt, eine Fördervorrichtung, die meist über einen Antrieb verfügt, um einen Kolben innerhalb des Behälters oder eine Förderpumpe zu betätigen, eine Düse zur Verteilung des Produkts und eine Hochspannungsquelle mit zugehöriger Hochspannungselektrode, um die Substanz bzw. deren Partikel und Tröpfchen elektrostatisch aufzuladen.

Alle genannten Auftragsmethoden haben den Nachteil, dass die kosmetische Substanz oft ungleichmäßig auf die Haut aufgetragen wird. Der Auftrag von Hand lässt sich nur ungenau dosieren und kontrollieren. Die verfügbaren Sprühgeräte geben die Substanz mit im Wesentlichen konstanter Ausbringungsmenge ab, was - bei einer ungleichmäßigen Bewegung oder einer falschen Ausrichtung des Behälters relativ zu einem Hautabschnitt - ebenfalls zu einer ungleichmäßigen Verteilung der Substanz führt. Mithin entstehen Bereiche auf der Haut, in denen zu viel, zu wenig oder gar keine Substanz aufgetragen worden ist. Letztere kann damit aber nicht optimal wirken, insbesondere dann, wenn es auf die richtige Dosierung ankommt.

Beim Auftrag kosmetischer Substanzen, insbesondere bei Sonnenschutz-Produkten, Selbstbräunern, Insektenschutzmitteln, Lotionen u.dgl., wird überdies oft - bewusst oder unbewusst - Material gespart, weil die Produkte relativ teuer sind. Bei Substanzen, die in regelmäßigen Abständen oder zur Erhaltung einer Heil- oder Schutzwirkung wiederholt aufgetragen werden müssen, wird die Einhaltung des wiederholten Auftragens oft vergessen. Individuelle Anpassungen beim Auftragen der kosmetischen Substanz auf die Haut einer Person sind nicht möglich.

EP 3 375 323 A1 offenbart eine Vorrichtung zum elektrostatischen Aufbringen einer Beschichtung auf die Haut einer Person, mit einer Abstandsmesseinheit zum Messen des Abstands zwischen der Haut und der Vorrichtung und mit einer Abstands-Entscheidungseinheit zum Entscheiden, ob der von der Abstandsmesseinheit ermittelte Abstand für das elektrostatische Sprühen geeignet ist. Eine Abstandsbenachrichtigungseinheit informiert den Benutzer über die Entscheidung der Abstandsentscheidungseinheit. Die Vorrichtung umfasst ferner eine Winkelmesseinheit zur Messung des Winkels zwischen der elektrostatischen Sprührichtung und der Haut.

Ziel der Erfindung ist es, ein nicht-therapeutisches Verfahren und ein Gerät zum Auftragen einer kosmetischen Substanz auf die Haut einer Person zu entwickeln, welches die Nachteile des Standes der Technik überwindet und einen möglichst gleichmäßigen und für die jeweilige Person optimalen Auftrag einer kosmetischen Substanz ermöglicht. Das Verfahren soll ferner individuelle Anpassungen vornehmen können und persönlichen Bedürfnissen gerecht werden. Es soll einfach aufgebaut und kostengünstig umsetzbar sein.

Hauptmerkmale der Erfindung sind in den Ansprüchen 1 und 18 angegeben. Ausgestaltungen sind Gegenstand der Ansprüche 2 bis 17 und 19.

Als Lösung schlägt die Erfindung ein nicht-therapeutisches Verfahren zum Auftragen einer kosmetischen Substanz auf die Haut einer Person vor, wobei das Verfahren unter Verwendung eines Gerätes zum elektrostatischen Versprühen der kosmetischen Substanz ausgeführt wird.

Das Gerät hat einen Behälter für die kosmetische Substanz, eine Düsenanordnung zum Versprühen der kosmetischen Substanz, eine Fördervorrichtung zum Fördern der kosmetischen Substanz von dem Behälter zu der Düsenanordnung, eine Hochspannungsquelle, einen Prozessor zum Verarbeiten von Messwerten, Daten und/oder Referenzwerten, einen Speicher zum Speichern der Messwerte, Daten und/oder Referenzwerte, eine Sende- und Empfangseinheit zum Übertragen der Messwerte, Daten und/oder Referenzwerte an eine externe Datenverarbeitungsvorrichtung und/oder zum Empfangen von weiteren Messwerten, Daten und/oder Referenzwerten von der externen Datenverarbeitungsvorrichtung.

Das Verfahren weist folgende Schritte auf:
▪ die Messwerte, weitere Messwerte, persönliche Daten der Person, weitere externe Daten und/oder Referenzwerte werden erfasst und/oder ermittelt und an den Prozessor übermittelt,
▪ der Prozessor verarbeitet die Messwerte, die weiteren Messwerte, die persönlichen Daten, die weiteren externen Daten und/oder die Referenzwerte zu wenigstens einem Ergebnis,
▪aus den Messwerten, den weiteren Messwerten, den persönlichen Daten der Person, den weiteren externen Daten und/oder den von dem Prozessor berechneten Ergebnissen wird eine Information über eine für die Person geeignete kosmetische Substanz generiert,
▪wobei über die Datenverarbeitungsvorrichtung oder über das Gerät selbst Informationen an die Person abgegeben werden, die das Gerät verwendet oder verwenden will, wobei sich diese Informationen auf die kosmetische Substanz beziehen,
▪ der Prozessor variiert anhand der Ergebnisse die Ausbringungsmenge der zu versprühenden kosmetischen Substanz aus dem Gerät.

Damit ist es möglich, mit dem Gerät einen weitgehend gleichmäßigen und für die jeweilige Person optimalen Auftrag der kosmetischen Substanz zu erreichen. Sämtliche Bereiche der zu behandelnden Haut können vollständig und mit einer stets ausreichenden und gleichmäßigen Menge an kosmetischer Substanz beschichtet werden, so dass letztere optimal die gewünschte oder vorgesehene Wirkung entfalten kann. Das Verfahren ist einfach strukturiert und lässt sich mit einfachen Mitteln kostengünstig umsetzen.

Erfindungsgemäß variiert der Prozessor die Ausbringungsmenge der zu versprühenden kosmetischen Substanz durch Regelung der Fördervorrichtung. Diese kann beispielsweise - als Förderpumpe ausgebildet - schneller oder langsamer betrieben werden. Treibt die Fördervorrichtung einen Kolben im Behälter für die kosmetische Substanz an, kann dieser schneller oder langsamer bewegt werden.

Ergänzend oder alternativ kann der Prozessor die Ausbringungsmenge der zu versprühenden kosmetischen Substanz durch Regelung der Hochspannungsquelle variieren. Hierdurch könnte zudem die Tröpfchengröße und -verteilung innerhalb der versprühten kosmetischen Substanz angepasst werden.

Eine weitere Ausgestaltung des erfindungsgemäßen Verfahrens sieht vor, dass der Prozessor die Ausbringungsmenge der zu versprühenden kosmetischen Substanz durch Beeinflussung der Düsenanordnung variiert. Dies kann beispielsweise dadurch erfolgen, dass der Düsenquerschnitt, die Düsenanordnung, die Düsenausrichtung oder die Zahl der zugeschalteten Düsen verändert werden.

Eine weitere Verbesserung des Auftrags der kosmetischen Substanz wird erreicht, indem die Variierung der Ausbringungsmenge der zur versprühenden kosmetischen Substanz anhand der Ergebnisse in Echtzeit erfolgt. Änderungen in den Messwerten, den weiteren Messwerten, den persönliche Daten der Person, den weitere externe Daten und/oder den Referenzwerte werden sofort berücksichtigt und die Ausbringungsmenge entsprechend angepasst.

In einer bevorzugten Ausführungsform hat das Gerät wenigstens einen Sensor, wobei die Messwerte von dem wenigstens einen Sensor während des Auftragens der kosmetischen Substanz auf die Haut der Person erfasst werden. Auch hierdurch kann die Ausbringungsmenge an kosmetischer Substanz an die individuellen Gegebenheiten und Bedürfnisse der Person, die das Gerät verwendet, angepasst werden.

Zweckmäßig erfasst der wenigstens eine Sensor eine Geschwindigkeit, eine Beschleunigung, eine Verzögerung und/oder eine Neigung des Gerätes.

Beispielweise wird in einer Ausbildung der Erfindung die Ausbringungsmenge der zu versprühenden kosmetischen Substanz in Abhängigkeit von der Geschwindigkeit des Gerätes variiert, wobei die Ausbringungsmenge der zu versprühenden kosmetischen Substanz bei steigender Geschwindigkeit erhöht wird und wobei die Ausbringungsmenge der zu versprühenden kosmetischen Substanz bei sinkender Geschwindigkeit vermindert wird.

Die Bewegungsgeschwindigkeit des Sprühgerätes wird mithin über einen oder mehrere verbaute Sensoren erfasst. Die ermittelten Messwerte werden an den Prozessor weitergeleitet, der die Messwerte verarbeitet und Bewegungsdaten errechnet. In Abhängigkeit dieser Bewegungsdaten wird die optimale Sprühmenge variiert, auch bei Geschwindigkeitswechseln und vorzugsweise in Echtzeit.

In einer alternativen oder ergänzenden Ausgestaltung der Erfindung wird die Ausbringungsmenge der zu versprühenden kosmetischen Substanz in Abhängigkeit von einer Beschleunigung und/oder einer Verzögerung des Gerätes variiert wird. Hierbei wird die Ausbringungsmenge der zu versprühenden kosmetischen Substanz bei einer Beschleunigung des Gerätes erhöht, während die Ausbringungsmenge der zu versprühenden kosmetischen Substanz bei einer Verzögerung des Gerätes vermindert wird.

In einer weiteren Ausführungsform der Erfindung wird die Ausbringungsmenge der zu versprühenden kosmetischen Substanz in Abhängigkeit von einer Neigung des Gerätes relativ zu einem Referenzpunkt variiert wird, wobei der Referenzpunkt eine Horizontale, eine Vertikale oder ein Bezugspunkt an oder auf der Person ist. Hierbei ist es zweckmäßig, wenn die Ausbringungsmenge der zu versprühenden kosmetischen Substanz bei größer werdender Neigung des Gerätes relativ zu dem Referenzpunkt erhöht wird und wenn die Ausbringungsmenge der zu versprühenden kosmetischen Substanz bei kleiner werdender Neigung des Gerätes relativ zu dem Referenzpunkt vermindert wird.

Bei allen drei Varianten - ob einzeln oder in Kombination - wird auf die Haut der Person, welche das Gerät verwendet, die stets richtige Schichtstärke an kosmetischer Substanz aufgetragen. Hierbei kann es sich beispielsweise um ein Sonnenschutzmittel handeln. Die Haut ist stets mit dem korrekten Sonnenschutzfaktor optimal geschützt.

In einer vorteilhaften Weiterbildung des erfindungsgemäßen Verfahrens kann der wenigstens eine Sensor die Spannung an der Hochspannungsquelle des Gerätes erfassen.

Bevorzugt wird für jede Art von Messwert ein separater Sensor vorgesehen, wobei für einen Messwert auch mehrere Sensoren eingesetzt werden können, deren Messwerte sich zu einer Information ergänzen.

Für die Erfassung der weiteren Messwerte kann wenigstens ein weiterer Sensor vorgesehen sein, damit der Prozessor bei der Berechnung des wenigstens einen Ergebnisses die weiteren Messwerte berücksichtigen kann.

Der wenigstens eine weitere Sensor erfasst z.B. die Umgebungstemperatur, die relative Luftfeuchtigkeit, den Luftdruck, die geografische Höhe, die geographische Position des Gerätes und dergleichen.

Der wenigstens eine weitere Sensor kann in dem Gerät selbst untergebracht sein. Er kann aber auch der externen Datenverarbeitungsvorrichtung zugeordnet und dort verbaut sein. In diesem Fall werden die weiteren Messwerte von der externen Datenverarbeitungsvorrichtung über die Sende- und Empfangseinheit des Gerätes an den Prozessor übermittelt.

Eine Weiterbildung der Erfindung sieht vor, dass der Prozessor bei der Berechnung des wenigstens einen Ergebnisses die persönlichen Daten der Person berücksichtigt. Dadurch lässt sich das Verfahren zum Auftragen der kosmetischen Substanz weiter personalisieren und an die individuellen Bedürfnisse der Person anpassen.

Die persönlichen Daten der Person werden bevorzugt in die externe Datenverarbeitungsvorrichtung, z.B. einen Computer, ein Tablet oder ein Smartphone, eingegeben, die mit einer entsprechenden Software versehen ist. Die persönlichen Daten können aber auch von der externen Datenverarbeitungsvorrichtung anhand einer Datenbank, im Internet, einer sonstigen Datenquelle, o.dgl. ermittelt werden.

Die persönlichen Daten der Person werden von der externen Datenverarbeitungsvorrichtung über die Sende- und Empfangseinheit des Gerätes an den Prozessor übermittelt, so dass dieser bei Bedarf die Ausbringungsmenge der zu versprühenden kosmetischen Substanz aus dem Gerät anpassen bzw. variieren kann.

Die persönlichen Daten der Person können sich beispielsweise auf das Geschlecht, das Alter, die Körpergröße, das Gewicht, den Hauttyp, die Hautfarbe, die Hautklasse, die Hautempfindlichkeit, die Nationalität, die Eigenschutzzeit (Erythemzeit), u.dgl. beziehen.

Der Prozessor kann ferner in einer noch anderen Ausführungsform der Erfindung bei der Berechnung des wenigstens einen Ergebnisses die weiteren externen Daten berücksichtigen.

Die weiteren externen Daten werden in die externe Datenverarbeitungsvorrichtung eingegeben oder von dieser anhand einer Datenbank, im Internet, einer sonstigen Datenquelle, o.dgl. ermittelt und anschließend von der externen Datenverarbeitungsvorrichtung über die Sende- und Empfangseinheit des Gerätes an den Prozessor übermittelt.

Die weiteren externen Daten können die Jahreszeit, die Tageszeit, die Uhrzeit, das Datum, das Land, in dem sich die Person befindet, der geografische Ort, an dem sich die Person befindet, die Umgebungstemperatur, die relative Luftfeuchtigkeit, der Luftdruck, die geografische Höhe, ein UV-Index, die örtliche UV-Belastung, u.dgl. sein.

Der Bezug von UV-Indexwerten kann aus öffentlich verfügbaren Datenbanken erfolgen. Die Werte können aber auch aus einem eingebauten UV-Sensor im Gerät ermittelt werden.

In einer weiteren wichtigen Ausführungsform des erfindungsgemäßen Verfahrens vergleicht der Prozessor bei der Berechnung des wenigstens einen Ergebnisses die Messwerte, die weiteren Messwerte, die persönlichen Daten und/oder die weiteren externen Daten mit Referenzwerten. Diese können beispielsweise als Grenzwerte ausgestaltet sein, so dass bestimmte Parameter nicht über- oder unterschritten werden und die Ausbringungsmenge an kosmetischer Substanz entsprechend angepasst werden kann.

Die Referenzwerte sind bevorzug in dem Speicher abgelegt.

Die Referenzwerte können aber auch ergänzend oder alternativ in die externe Datenverarbeitungsvorrichtung eingegeben oder von dieser anhand einer Datenbank, im Internet, einer sonstigen Datenquelle, o.dgl. ermittelt werden.

Die Referenzwerte werden von der externen Datenverarbeitungsvorrichtung über die Sende- und Empfangseinheit des Gerätes an den Prozessor übermittelt.

In einer vorteilhaften Variante des Verfahrens wird aus den Messwerten des wenigstens einen Sensors, den weiteren Messwerten des wenigstens einen weiteren Sensors, den persönlichen Daten der Person, der weiteren externen Daten und/oder den von dem Prozessor ermittelten Ergebnissen ein Bewegungsprofil des Gerätes und/oder der Person, die das Gerät führt, ermittelt.

Dieses Bewegungsprofil wird vorzugsweise von dem Prozessor berechnet. Es kann aber auch von der externen Datenverarbeitungsvorrichtung berechnet und anschließend an das Gerät und den Prozessor übermittelt werden.

Jedes Bewegungsprofil ist zweckmäßig einer Person oder eine Personengruppe zugeordnet, wobei die Zuordnung anhand der persönlichen Daten und/oder anhand der externen Daten erfolgt.

Jedes oder ausgewählte Bewegungsprofile und/oder die Zuordnung eines Bewegungsprofils zu einer Person werden in dem Speicher abgelegt. Man kann die Profile aber auch extern in der externen Datenverarbeitungsvorrichtung speichern.

Damit ist es möglich, eine Person, namentlich einen Benutzer des Gerätes, zu identifizieren und die Ausbringungsmenge der kosmetischen Substanz entsprechend einzustellen bzw. anzupassen.

Eine wichtige Weiterbildung des erfindungsgemäßen Verfahrens sieht daher vor, dass das aktuell ermittelte oder berechnete Bewegungsprofil mit den gespeicherten Bewegungsprofilen verglichen wird und dass im Falle einer zumindest teilweisen Übereinstimmung mit einem gespeicherten Bewegungsprofil die Ausbringungsmenge der zu versprühenden kosmetischen Substanz aus dem Gerät angepasst oder variiert wird.

Der Vergleich der Bewegungsprofile wird von dem Prozessor und/oder von der externen Datenverarbeitungsvorrichtung durchgeführt wird, wobei festzulegen ist, zu welchem Grad oder in welchen Bereich das ermittelte Bewegungsprofil mit einem gespeicherten Bewegungsprofil übereinstimmen muss.

Eine vorteilhafte Weiterbildung der Erfindung sieht vor, dass aus den Messwerten, den weiteren Messwerten, den persönlichen Daten der Person, der weiteren externen Daten, den von dem Prozessor berechneten Ergebnissen und/oder dem Bewegungsprofil des Gerätes eine Information über den Hauttyp und/oder das Hautprofil der Person generiert wird.

Damit besteht nicht nur die Möglichkeit die individuell optimale Ausbringungsmenge für die kosmetische Substanz zu ermitteln und zu realisieren. Es besteht vielmehr die Möglichkeit der Person, welche das Gerät verwenden will, individuelle Empfehlungen in Bezug auf die Auswahl der kosmetischen Substanz zu geben.

Die Person kann für die Bestimmung des Hauttyps und/oder des Hautprofils weitere Informationen oder Angaben in die externe Datenverarbeitungsvorrichtung eingeben. Diese ist hierzu mit einer entsprechenden Software oder App versehen. Zu den weiteren Informationen können auch Bilder von der Person, vom Gesicht der Person oder von Hautabschnitten der Person zählen, die in die externe Datenverarbeitungsvorrichtung geladen werden.

Die Informationen und Angaben der Person werden von der externen Datenverarbeitungsvorrichtung analysiert und/oder ausgewertet und zu persönlichen Informationen verarbeitet.

Damit ist nicht nur eine individuelle Anpassung der Ausbringungsmenge an kosmetischer Substanz möglich. Das Verfahren gibt über die Datenverarbeitungsvorrichtung oder über das Gerät selbst Informationen an die Person ab, die das Gerät verwendet oder verwenden will. Diese Informationen können sich auf die kosmetische Substanz oder auf die Verwendungsweise des Gerätes beziehen oder auf die Häufigkeit und die Intervalle der Anwendung des Gerätes mit der jeweiligen kosmetischen Substanz.

Eine weitere Ausführungsform der Erfindung sieht vor, dass aus den Messwerten, den weiteren Messwerten, den persönlichen Daten der Person, der weiteren externen Daten, den von dem Prozessor berechneten Ergebnissen und/oder dem Bewegungsprofil des Gerätes eine Information über einen für die Person geeigneten Sonnenschutzfaktor generiert wird. Eine noch andere Ausführungsform der Erfindung sieht vor, dass aus den Messwerten, den weiteren Messwerten, den persönlichen Daten der Person, der weiteren externen Daten, den von dem Prozessor berechneten Ergebnissen und/oder dem Bewegungsprofil des Gerätes eine Information über eine für die Person geeignete kosmetische Substanz generiert wird.

Eine Weiterbildung der Erfindung sieht ferner vor, dass aus den Messwerten, den weiteren Messwerten, den persönlichen Daten der Person, der weiteren externen Daten, den von dem Prozessor berechneten Ergebnissen und/oder dem Bewegungsprofil des Gerätes eine Zeit-Information für die Person generiert wird.

Das Gerät und/oder die Datenverarbeitungsvorrichtung können dadurch die Person, welche das Gerät verwendet hat an eine erneute oder wiederholte Anwendung erinnern, so dass die kosmetische Substanz stets optimal aufgetragen und optimal wirken kann.

Die Information über den Hauttyp und/oder das Hautprofil der Person, die persönlichen Informationen, der geeignete Sonnenschutzfaktor, die geeignete kosmetische Substanz und/oder die Zeit-Information werden bevorzugt von dem Prozessor und/oder von der externen Datenverarbeitungsvorrichtung bestimmt.

Mit besonderem Vorteil werden ferner die Information über den Hauttyp und/oder das Hautprofil der Person, die persönlichen Informationen, der geeignete Sonnenschutzfaktor, die geeignete kosmetische Substanz und/oder die Zeit-Information von der externen Datenverarbeitungsvorrichtung über die Sende- und Empfangseinheit des Gerätes an den Prozessor übermittelt und zu den persönlichen Daten der Person und/oder zu den weiteren externen Daten hinzugefügt.

Dadurch kann das erfindungsgemäße Verfahren eine selbstlernende Diagnose durchführen und die Ausbringungsmenge der kosmetischen Substanz stets optimal auf die das Gerät jeweils verwendende Person anpassen. Es handelt sich mithin um ein iterativ lernendes Verfahren.

Dementsprechend sieht eine weitere vorteilhafte Ausbildung der Erfindung vor, dass der Prozessor während der Verwendung des Gerätes anhand der Information über den Hauttyp und/oder das Hautprofil der Person, der persönlichen Informationen, des geeigneten Sonnenschutzfaktors, der Information über die geeignete kosmetische Substanz und/oder die Zeit-Information die Ausbringungsmenge der zu versprühenden kosmetischen Substanz aus dem Gerät anpasst oder variiert.

Um die Anwendung und den Auftrag der kosmetischen Substanz weiter zu individualisieren, zu personalisieren und/oder zu optimieren, sieht das erfindungsgemäße Verfahren vor, dass die Person nach der Verwendung des Geräts Antworten auf Fragen in die externe Datenverarbeitungsvorrichtung eingeben muss.

Die Antworten der Person werden von der externen Datenverarbeitungsvorrichtung analysiert und/oder ausgewertet und zu weiteren persönlichen Informationen verarbeitet, wobei die weiteren persönlichen Informationen von der externen Datenverarbeitungsvorrichtung über die Sende- und Empfangseinheit des Gerätes an den Prozessor übermittelt und zu den persönlichen Daten der Person hinzugefügt werden. Auf der externen Datenverarbeitungsvorrichtung ist auch hierzu eine entsprechende Software oder App installiert.

Eine weitere Ausbildung der Erfindung sieht vor, dass beim Einschalten des Geräts ein Wechselstromimpuls erzeugt und ein von der Person reflektiertes Signal von einem Sensor oder einem weiteren Sensor erfasst wird.

Dadurch kann das Gerät von mehr als einer Person verwendet werden, beispielsweise innerhalb einer Familie oder unter Freunden. Sobald eine Person das Gerät in die Hand nimmt und einschaltet, wird der Wechselstromimpuls ausgelöst. Dessen "Echo" wird von der Person zurück in das Gerät gesendet und dort von einem geeigneten Sensor erfasst. Aus dieser Information lässt sich beispielsweise ein Körpervolumen errechnen, anhand dessen die Person identifiziert werden kann, ggf. unter Hinzuziehung von weiteren persönlichen Daten. Sobald die Person erkannt ist, können Nutzungsdaten automatisch und korrekt zugeordnet werden, um beispielsweise einen Anwendungs-Alarm neu zu setzen.

Das Gerät hat zweckmäßig ein Gehäuse, in dem der Behälter für die kosmetische Substanz, die Düsenanordnung zum Versprühen der kosmetischen Substanz, die Fördervorrichtung zum Fördern der kosmetischen Substanz von dem Behälter zu der Düsenanordnung, die Hochspannungsquelle, der Prozessor zum Verarbeiten von Messwerten, Daten und/oder Referenzwerten, der Speicher zum Speichern der Messwerte, Daten und/oder Referenzwerte und die Sende- und Empfangseinheit zum Übertragen der Messwerte, Daten und/oder Referenzwerte an eine externe Datenverarbeitungsvorrichtung und/oder zum Empfangen von weiteren Messwerten, Daten und/oder Referenzwerten von der externen Datenverarbeitungsvorrichtung untergebracht sind.

Der Prozessor, der Speicher und oder der wenigstens eine weitere Sensor können ergänzend oder alternativ in der externen Datenverarbeitungsvorrichtung untergebracht sein. Damit könnte das Gerät insgesamt kompakter ausgebildet werden.

Die externe Datenverarbeitungsvorrichtung ist bevorzugt ein Computer, ein Tablet, ein Smartphone, eine Konsole, oder dergleichen.

In einer noch anderen Ausführungsform des erfindungsgemäßen Verfahrens legt die externe Datenverarbeitungsvorrichtung die Messwerte, die weiteren Messwerte, die persönlichen Daten der Person, die weiteren externen Daten, das wenigstens eine von dem Prozessor berechnete Ergebnis und/oder das Bewegungsprofil des Gerätes in einer Datenbank ab. Diese kann in einer Cloud gespeichert sein.

Aus diesen Daten kann das Verfahren erkennen, dass z.B. viele vergleichbare Personen (z.B. mit gleichem Hauttyp, am gleichen Ort, bei vergleichbarer UV-Belastung) sich nicht ausreichend eingecremt haben. Diese Information wird dann lernend an die gesamte Personengruppe weitergegeben und das jeweilige Gerät wird entsprechend der individuellen Person verwendet, d.h. es wird - unter Berücksichtigung der Messwerte, der weiteren Messwerte, der persönlichen Daten, der weiteren externen Daten und/oder der Referenzwerte - die für die jeweilige Person optimale Ausbringungsmenge an kosmetischer Substanz generiert.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:

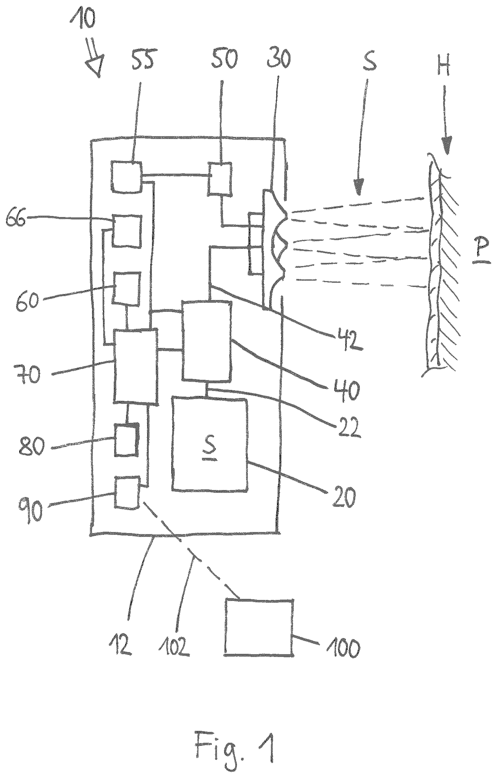

- Fig. 1: eine schematische Darstellung eines Geräts für die Durchführung des erfindungsgemäßen Verfahren.

Das in Fig. 1 rein schematisch dargestellte Gerät 10 ist zum Auftragen einer kosmetischen Substanz S auf die Haut H einer (nicht näher dargestellten) Person P vorgesehen. Die kosmetische Substanz S kann beispielsweise ein Hautpflegeprodukt, eine Lotion, ein Sonnenschutzmittel, ein Selbstbräuner, ein Insektenschutzmittel, u.dgl. sein. Die Person ist ein Mann, eine Frau, ein Kind, egal in welchem Alter.

Das Gerät 10 ist als elektrostatisches Sprühgerät ausgebildet. Es hat in einem Gehäuse 12 einen Behälter 20 für die kosmetische Substanz S, eine Düsenanordnung 30 zum Versprühen der kosmetischen Substanz S, eine Fördervorrichtung 40, zum Fördern der kosmetischen Substanz S von dem Behälter 20 zu der Düsenanordnung 30 sowie eine Hochspannungsquelle 50. Zwischen dem Behälter 20, der Fördervorrichtung 40 und der Düsenanordnung 30 ist ein Schlauchsystem 22, 42 angeordnet. Die Hochspannungsquelle 50 hat eine oder mehrere (nicht näher dargestellte) Elektrode(n) für die elektrostatische Zerstäubung in der Düsenanordnung 30.

In dem Gehäuse befindet sich ferner ein Prozessor 70 zum Verarbeiten von Messwerten, Daten und/oder Referenzwerten, ein Speicher 80 zum Speichern der Messwerte, Daten und/oder Referenzwerte, sowie eine Sende- und Empfangseinheit 90 zum Übertragen der Messwerte, Daten und/oder Referenzwerte an eine externe Datenverarbeitungsvorrichtung 100 und/oder zum Empfangen von weiteren Messwerten, Daten und/oder Referenzwerten von der externen Datenverarbeitungsvorrichtung 100.

Die externe Datenverarbeitungsvorrichtung ist bevorzugt ein Computer, ein Tablet, ein Smartphone, eine Konsole, o.dgl., die über eine Funkstrecke 102 mit der Sende- und Empfangseinheit 90 des Geräts 10 verbunden ist.

Das Gerät 10 hat ferner eine Energieversorgung 55, beispielsweise eine auswechselbare Batterie oder einen Akku, wobei der Akku über ein externes (nicht gezeigtes) Ladegerät bei Bedarf aufgeladen werden kann. Die Energieversorgung 55 versorgt die Fördervorrichtung 40, die Hochspannungsquelle 50 sowie den Prozessor 70 mit elektrischer Energie.

Über wenigstens einen (nicht dargestellten) Schalter oder Taster kann das Gerät 10 in Betrieb gesetzt werden. Gleichfalls nicht gezeigte akustische und/oder optische Signalgeber informieren - bei Bedarf - über den Betriebszustand des Gerätes 10 sowie über den Füllstand in dem Behälter 20. Bei Bedarf kann das Gerät 10 auch mit einem (gleichfalls nicht gezeigten) Display versehen sein.

Das erfindungsgemäße Verfahren sieht in einer Ausführungsform vor, dass die Messwerte erfasst und/oder ermittelt und an den Prozessor 70 übermittelt werden. Anschließend verarbeitet der Prozessor 70 mittels einer geeigneten Software die Messwerte zu wenigstens einem Ergebnis. Anhand dieses Ergebnisses variiert der Prozessor 70 die Ausbringungsmenge der zu versprühenden kosmetischen Substanz S aus dem Gerät 10.

Die Variierung der Ausbringungsmenge kann dadurch erfolgen, dass der Prozessor 70 die Fördervorrichtung 40 regelt, beispielsweise indem die Fördervorrichtung 40 mehr oder weniger von der kosmetischen Substanz aus dem Behälter 20 zu der Düsenanordnung 30 fördert. Geregelt wird vorzugsweise die Fördergeschwindigkeit. Man kann aber auch das Fördervolumen ändern bzw. anpassen.

Der Prozessor 70 kann - ergänzend oder alternativ - die Ausbringungsmenge der zu versprühenden kosmetischen Substanz S durch Regelung der Hochspannungsquelle 50 variieren, indem beispielsweise die an den Elektroden anliegende Hochspannung verändert wird.

Denkbar ist auch, dass der Prozessor 70 die Ausbringungsmenge der zu versprühenden kosmetischen Substanz S dadurch variiert, dass die Düsenanordnung 30 verändert wird. Beispielsweise lassen sich die Düsenquerschnitte beeinflussen oder einzelne Düsen werden hinzu oder abgeschaltet. Denkbar ist ferner, den Sprühwinkel zu beeinflussen.

Von Vorteil ist, wenn die Variierung der Ausbringungsmenge der zur versprühenden kosmetischen Substanz S anhand der Ergebnisse, die der Prozessor berechnet hat, in Echtzeit erfolgt. Auf diese Weise werden Änderungen in den ermittelten Messwerten sofort berücksichtig und die Ausbringungsmenge entsprechend angepasst, was sich günstig auf die Verteilung der kosmetischen Substanz auf der Haut H der Person P auswirkt.

Das Gerät 10 kann so ausgebildet sein, dass neben den Messwerten auch weitere Messwerte, persönliche Daten der Person P, weitere externe Daten und/oder Referenzwerte bei der Berechnung des wenigstens einen Ergebnisses berücksichtigt werden.

In dem Gerät 10 kann für die Erfassung von Messwerten wenigstens ein Sensor 60 vorgesehen. Dieser wird von dem erfindungsgemäßen Verfahren derart eingesetzt, dass die Messwerte während des Auftragens der kosmetischen Substanz S auf die Haut H der Person P erfasst werden.

Beispielsweise erfasst der Sensor 60, während das Gerät 10 von der Person P bewegt wird, eine Geschwindigkeit. Diese Messwerte werden an den Prozessor 70 übermittelt und ausgewertet. Auf diese Weise ist es möglich, die Ausbringungsmenge der zu versprühenden kosmetischen Substanz S in Abhängigkeit von der Geschwindigkeit des Gerätes 10 zu variieren, d.h. die Ausbringungsmenge wird an die Bewegung des Gerätes 10 durch die Person P angepasst. Bei steigender Geschwindigkeit kann die Ausbringungsmenge über den Prozessor 70 erhöht werden, während die Ausbringungsmenge der zu versprühenden kosmetischen Substanz S bei sinkender Geschwindigkeit vermindert wird.

Die Geschwindigkeit kann über einen Sensor 60 erfasst werden. Es können aber auch mehrere Sensoren, vorzugsweise drei Sensoren 60 eingesetzt werden, um die Geschwindigkeit des Gerätes 10 in allen drei Raumrichtungen erfassen zu können. Die ermittelten Messwerte werden an den Prozessor 70 weitergeleitet, der die Messwerte verarbeitet und Bewegungsdaten errechnet (Ergebnisse). In Abhängigkeit dieser Bewegungsdaten wird die optimale Sprühmenge berechnet und die Ausbringungsmenge entsprechend variiert, vorzugsweise in Echtzeit.

Damit ist es möglich, eine bewegungsabhängige, variable Ausbringungsmenge der kosmetischen Substanz S zur Erzielung einer erwünschten Schichtstärke zu generieren.

Bei Sonnenschutzmitteln beispielsweise setzt der für die jeweilige Person P richtige Schutz einerseits den richtigen UV-Filteranteil in der kosmetischen Substanz S voraus, der dann andererseits in der richtigen Schichtstärke auf die Haut H aufgetragen werden muss. Zu wenig Sonnenschutz "verdünnt" den Sonnenschutz (Beispiel: ein Sonnenschutzmittel mit dem Sonnenschutzfaktor SPF50 zu dünn aufgetragen schützt nur wie ein Sonnenschutzmittel mit dem Sonnenschutzfaktor SPF30). Hinzu kommt, dass Verbraucher im Schnitt 80 bis 90 % zu wenig an Sonnenschutzmittel auf die Haut auftragen, so dass auch hierdurch kein optimaler Sonnenschutz gewährleistet ist.

Das Aufbringen der richtigen Schichtstärke mit einem Sprühgerät erfordert bei handgehaltenen Geräten ein exaktes Zusammenspiel von Bewegungsgeschwindigkeit der geräteführenden Hand und der Ausbringungsmenge. Gängige Sprühgeräte haben nur eine konstante Ausbringungsmenge. Weil aber die Bewegungsgeschwindigkeit des Arms der Person P, welche das Sonnenschutzmittel aufbringt, immer unterschiedlich schnell ist, und weil auch die Bewegungsmotorik der Person P beim Sprühvorgang unterschiedlich ist, führt eine konstante Ausbringungsmenge zu unterschiedlichen Schichtstärken.

Das erfindungsgemäße Verfahren sieht hingegen vor, dass die Bewegungsgeschwindigkeit des Gerätes 10 über die verbauten Sensoren 60 erfasst wird. Die ermittelten Messwerte werden an den Prozessor 70 übermittelt, der die Messwerte analysiert und Bewegungsdaten errechnet. Anhand dieser Ergebnisse, d.h. in Abhängigkeit von der

Bewegungsgeschwindigkeit des Gerätes 10, wird die Ausbringungsmenge der kosmetischen Substanz S variiert, so dass in jedem Abschnitt der Haut H der Person P stets die optimale Menge an Substanz S aufgebracht wird. Die Variierung der Ausbringungsmenge kann dadurch erfolgen, dass über den Prozessor 70 die Fördervorrichtung 40, die Hochspannungsquelle 50 und/oder die Düsenanordnung 30 geregelt und/oder verändert werden.

In einer alternativen oder ergänzenden Ausgestaltung des Verfahrens wird die Ausbringungsmenge der zu versprühenden kosmetischen Substanz S in Abhängigkeit von einer Beschleunigung und/oder einer Verzögerung des Gerätes 10 variiert wird. Beispielsweise wird die Ausbringungsmenge bei einer Beschleunigung des Gerätes 10 erhöht, während die Ausbringungsmenge bei einer Verzögerung des Gerätes 10 vermindert wird.

Möglich ist auch, die Ausbringungsmenge der zu versprühenden kosmetischen Substanz S in Abhängigkeit von einer Neigung des Gerätes 10 relativ zu einem Referenzpunkt zu variieren, wobei der Referenzpunkt eine Horizontale, eine Vertikale oder ein Bezugspunkt an oder auf der Person P ist. Beispielsweise ist es zweckmäßig, wenn die Ausbringungsmenge bei größer werdender Neigung des Gerätes 10 relativ zu dem Referenzpunkt erhöht wird, während die Ausbringungsmenge bei kleiner werdender Neigung des Gerätes 10 relativ zu dem Referenzpunkt vermindert wird.

Weil die Haut H von Personen P individuell unterschiedlich ist und der geographische Standort von Verbrauchern unterschiedlich sein kann, sind auch die idealen Werte für einen Sonnenschutzfaktor von Verbraucher zu Verbraucher unterschiedlich. Bei fixen, konkreten Sonnenschutzfaktoren ist der Schutz bei jedem Nutzer damit nur unterschiedlich ausreichend.

Das erfindungsgemäße Verfahren bietet die Möglichkeit, über die Variierung der Ausbringungsmenge und damit der Schichtstärke der kosmetischen Substanz S den Auftrag individuell an unterschiedlichste Personen P anzupassen, indem weitere Messwerte, persönliche Daten der Person P, weitere externe Daten und/oder Referenzwerte erfasst und/oder ermittelt und an den Prozessor 70 übermittelt werden.

Dadurch lässt sich der z.B. tatsächlich aufgebrachte Sonnenschutzfaktor eines Sonnenschutzmittels personalisiert steuern, indem beispielsweise von einem Sonnenschutzmittel mit dem Sonnenschutzfaktor 50 bewusst zu wenig aufgetragen wird, d.h. der Sonnenschutzfaktor 50 wird auf 30 "verdünnt".

Die personenindividuelle, ideale Ausbringungsmenge kann mithin über die gelernten, personenbezogenen Diagnosedaten und über die Ziel-Schichtstärke bestimmt werden. Damit aber kann in dem Gerät z.B. nur eine einzige Sorte Sonnenschutzmittel vorgehalten werden, das für verschiedenste Personen P verwendet wird. Beispielsweise wird das Gerät 10 mit einem Sonnenschutzmittel mit dem Sonnenschutzfaktor 50 gefüllt. Mit dem erfindungsgemäßen Verfahren kann diese Substanz S in unterschiedliche Sonnenschutzfaktoren "gestreckt" werden und damit einer Massen-Personalisierung zugänglich gemacht werden.

Für die Erfassung der weiteren Messwerte kann wenigstens ein weiterer Sensor 66 vorgesehen sein, damit der Prozessor 70 bei der Berechnung des wenigstens einen Ergebnisses die weiteren Messwerte berücksichtigen kann.

Der wenigstens eine weitere Sensor 66 erfasst z.B. die Umgebungstemperatur, die relative Luftfeuchtigkeit, den Luftdruck, die geografische Höhe, die geographische Position des Gerätes 10 und dergleichen.

Der wenigstens eine weitere Sensor kann in dem Gerät 10 selbst untergebracht sein. Er kann aber auch der externen Datenverarbeitungsvorrichtung 100 zugeordnet und dort verbaut sein. In diesem Fall werden die weiteren Messwerte von der externen Datenverarbeitungsvorrichtung 100 über die Sende- und Empfangseinheit 90 des Gerätes 10 an den Prozessor 70 übermittelt.

Der Prozessor 70 kann ferner bei der Berechnung des wenigstens einen Ergebnisses die persönlichen Daten der Person P berücksichtigen. Diese werden bevorzugt in die externe Datenverarbeitungsvorrichtung 100, z.B. einen Computer, ein Tablet oder ein Smartphone, eingegeben, die mit einer entsprechenden Software versehen ist und mit dem Gerät 10 über die Funkstrecke 102 in Verbindung steht. Die persönlichen Daten können aber auch von der externen Datenverarbeitungsvorrichtung 100 anhand einer Datenbank, im Internet, einer sonstigen Datenquelle, o.dgl. ermittelt werden.

Die persönlichen Daten der Person werden von der externen Datenverarbeitungsvorrichtung 100 über die Sende- und Empfangseinheit 90 des Gerätes 10 an den Prozessor 70 übermittelt, so dass dieser bei Bedarf die Ausbringungsmenge der zu versprühenden kosmetischen Substanz S aus dem Gerät 10 anpassen bzw. variieren kann.

Die persönlichen Daten der Person beziehen sich beispielsweise auf das Geschlecht, das Alter, die Körpergröße, das Gewicht, den Hauttyp, die Hautfarbe, die Hautklasse, die Hautempfindlichkeit, die Nationalität, die Eigenschutzzeit (Erythemzeit), u.dgl.

Weitere Anpassungen des Verfahrens, weitere Individualisierungen an die das Gerät 10 verwendende Person P und damit eine noch genauere Anpassung und Personalisierung der Ausbringungsmenge wird erreicht, wenn der Prozessor 70 bei der Berechnung des wenigstens einen Ergebnisses die weiteren externen Daten berücksichtigt.

Die weiteren externen Daten werden bevorzugt in die externe Datenverarbeitungsvorrichtung 100 eingegeben oder von dieser anhand einer Datenbank, im Internet, einer sonstigen Datenquelle, o.dgl. ermittelt. Anschließend werden die Daten von der externen Datenverarbeitungsvorrichtung 100 über die Sende- und Empfangseinheit 90 des Gerätes 10 an den Prozessor 72 übermittelt.

Die weiteren externen Daten können die Jahreszeit, die Tageszeit, die Uhrzeit, das Datum, das Land, in dem sich die Person befindet, der geografische Ort, an dem sich die Person befindet, die Umgebungstemperatur, die relative Luftfeuchtigkeit, der Luftdruck, die geografische Höhe, ein UV-Index, die örtliche UV-Belastung, u.dgl. sein.

Für eine weitere Individualisierung und Optimierung der Ausbringungsmenge an kosmetischer Substanz S aus dem Gerät 10 bei der Anwendung durch eine Person P kann der Prozessor 70 bei der Berechnung des wenigstens einen Ergebnisses die Messwerte, die weiteren Messwerte, die persönlichen Daten und/oder die weiteren externen Daten mit Referenzwerten vergleichen.

Diese Referenzwerte sind vorzugsweise Grenzwerte sein, die den einen oder andern Parameter nach oben und/oder unten begrenzen, so dass der vorgegebene Wert nicht über- oder unterschritten wird. Auf diese Weise lassen sich beispielsweise Temperaturbereiche oder Tageszeiten definieren, um die Ausbringungsmenge an kosmetischer Substanz S entsprechend anpassen zu können.

Die Referenzwerte sind bevorzug in dem Speicher abgelegt. Sie können aber auch in die externe Datenverarbeitungsvorrichtung 100 eingegeben oder von dieser anhand einer Datenbank, im Internet, einer sonstigen Datenquelle, o.dgl. ermittelt werden. Nach Festlegung der Referenzwerte werden diese von der externen Datenverarbeitungsvorrichtung 100 über die Sende- und Empfangseinheit 90 an das Gerät 10 bzw. an den Prozessor 70 übermittelt.

Mit der Ausbildung des erfindungsgemäßen Verfahrens lassen sich unterschiedlichste Anwendungen realisieren.

So ist es beispielsweise möglich, einen Nutzer, d.h. eine Person P zu identifizieren und die Ausbringungsmenge des Gerätes 10 entsprechend der erkannten Person anzupassen bzw. zu variieren.

Dies ist notwendig, wenn das Gerät 10 von mehr als einer Person P verwendet wird (z.B. innerhalb einer Familie, einer Gruppe oder unter Freunden). Folglich stellt sich die Aufgabe, die Nutzungsdaten automatisch dem individuellen Nutzer P richtig zuzuordnen, um etwa die Ausbringungsmenge anzupassen, einen Zeitalarm auszulösen für einen wiederholten Auftrag der kosmetischen Substanz S - beispielsweise eines Sonnenschutzmittels - oder um an den Wechsel des Behälters und damit der Substanz S zu erinnern. Ein händisches Zuweisen der Nutzungsdaten (etwa vor dem Einsprühvorgang oder danach) wäre unpraktisch und würde ohnehin oft vergessen.

In einer vorteilhaften Variante des Verfahrens wird daher aus den Messwerten des wenigstens einen Sensors 60, den weiteren Messwerten des wenigstens einen weiteren Sensors 66, den persönlichen Daten der Person, der weiteren externen Daten und/oder den von dem Prozessor ermittelten Ergebnissen ein Bewegungsprofil des Gerätes 10 und/oder der Person P, die das Gerät 10 führt, ermittelt. Dieses Bewegungsprofil wird vorzugsweise von dem Prozessor 70 berechnet. Es kann aber auch von der externen Datenverarbeitungsvorrichtung 100 berechnet und anschließend an das Gerät 10 und den Prozessor 70 übermittelt werden.

Jedes Bewegungsprofil ist zweckmäßig einer Person P oder eine Personengruppe zugeordnet, wobei die Zuordnung anhand der persönlichen Daten und/oder anhand der externen Daten erfolgt.

Damit ist das Gerät 10 in der Lage, beispielsweise während eines Eincreme-Vorgangs, die einzigartige Bewegungsmotorik einer Person P wie einen Fingerabdruck zu erfassen und zu identifizieren. Sind z.B. zwei Personen P unterschiedlich groß, dann legt das Gerät 10 bei jeder dieser Personen während des Einsprühens eine unterschiedliche lange Strecke im Raum zurück. Ferner wird das Gerät unterschiedlich gehalten, d.h. auch die Beschleunigung und die Neigung können unterschiedlich sein. Jede Person fährt damit beim Einsprühen mit dem Gerät 10 den Körper ab, wobei die Bewegung den Körperkonturen folgt. Durch Erfassung dieses Bewegungsprofils (über Geschwindigkeiten, Neigungen und Beschleunigungen) kann eine Körperform erfasst werden, durch die dann eine Personenidentifikation ermöglich ist. Je häufiger sich eine Person P einsprüht, desto genauer erfasst das Gerät die Körperform und das Motorik-Profil, so dass nach einem "Anlernen" des Gerätes 10 eine stets zuverlässige Nutzererkennung möglich ist.

Dies erlaubt es zudem, Nutzungsdaten automatisch einem identifizierten Nutzer zuzuordnen, sobald dieser das Gerät 10 bewegt oder seine persönlichen Daten eingegeben oder abgerufen hat.

Jedes oder ausgewählte Bewegungsprofile und/oder die Zuordnung eines Bewegungsprofils zu einer Person werden in dem Speicher 80 abgelegt. Man kann die Profile aber auch extern in der externen Datenverarbeitungsvorrichtung 100 speichern. Auf diese Weise lassen sich die Bewegungsprofile vergleichen, d.h. wenn eine Person beginnt sich einzusprühen, kann der Prozessor 70 die Person erkennen.

In einer weiteren Ausgestaltung kann das Gerät 10, sobald die Person P das Gerät 10 in die Hand nimmt und einschaltet, einen Wechselstromimpuls auslösen. Durch das von der Person reflektierte Signal lässt sich ein Körpervolumen errechnen. Wenn zwei Personen P unterschiedliche Körpergrößen und Gewichte haben, wird auch das Echo unterschiedliche Signale erzeugen. Mit diesen Signalen können dann von dem Gerät 10 die Personen P unterschieden werden.

Das erfindungsgemäße Verfahren ermöglicht ferner die Durchführung von Diagnosen und die individuelle Empfehlung einer kosmetischen Substanz S, beispielsweise in Bezug auf die Auswahl eines Sonnenschutzmittels mit einem bestimmten Sonnenschutzfaktor.

Endverbraucher wissen oft nicht, welcher Sonnenschutzfaktor für sie ideal ist. Typischerweise nehmen sie den nächstbesten Sonnenschutzfaktor, oder immer den gleichen, gewohnten Sonnenschutzfaktor. Sonnenbrandinzidenzen zeigen an, dass Endverbraucher hier Fehler bei der Auswahl machen.

In die richtige Auswahl des Sonnenschutzfaktors fließen verschiedene Parameter ein.

Zunächst muss die konkrete UV-Belastung vor Ort einfließen. Der Endverbraucher kann die UV-Belastung zwar von öffentlichen Einrichtungen erfahren (auch via Smartphone). Diese sind aber meist sehr ungenau, weil der UV-Index nicht flächendeckend erhoben wird (in Deutschland z.B. nur einmal pro Bundesland).

Als weiterer Parameter ist die höchst individuelle Eigenschutzzeit bzw. Erythemzeit der eigenen Haut zu berücksichtigen. Die Eigenschutzzeit kann der Endverbraucher schätzen, indem er sich über In-Augenscheinnahme einer von fünf Hauttypen-Klassen zuordnet. Diese Hauttypen stellen jedoch eine sehr starke Vereinfachung dar und lassen ohnehin keine abnormalen Eigenschutzzeiten zu (z.B. dunkle Haut, aber große Hautempfindlichkeit). Zugleich ist davon auszugehen, dass die tatsächliche UV-Belastung und die Eigenschutzzeit keinen linearen Zusammenhang haben.

Schließlich ist Sonnenschutzfaktor selber nur ein ungefährer Wert. Er wird für eine Formulierung per Standardverfahren erhoben, indem die Sonnenbrandzeit mit Sonnenschutz bei einer definierten Anzahl von Probanden ermittelt wird. Anschließend werden die Ergebnisse gewichtet und in einen Wert für den Sonnenschutzfaktor umgerechnet. Aufgrund der Durchschnittsbildung alleine sowie allgemeiner Schwierigkeiten der homogenen Auftragung ist damit dieser Wert keinesfalls genau.

Das erfindungsgemäße Verfahren schafft hier Abhilfe.

Durch die Berücksichtigung und Eingabe der weiteren Messwerte, der persönlichen Daten der Person P, der weiteren externen Daten und/oder der Referenzwerte kann über das Gerät 10 eine individuelle Bestimmung des eigenen Hautprofils durchgeführt werden, vorzugsweise auf der externen Datenverarbeitungsvorrichtung 100 und mittels einer darauf installieren Software (App).

Diese Software kann beispielsweise vor der Benutzung des Gerätes 10 Fragen stellen, die die Person P beantworten muss. Die Fragen können durch ein Bild der Person ergänzt werden, z.B. durch ein Foto vom Gesicht oder von ausgewählten empfindlichen Hautstellen. Die Software analysiert die eingegebenen Antworten und Bilder und erstellt daraus persönliche Daten, die anschließend von der externen Datenverarbeitungsvorrichtung 100 über die Sende- und Empfangseinheit 90 an das Gerät 10 und damit an den Prozessor 70 übermittelten werden.

Ergänzt werden können die persönlichen Daten durch den Bezug von UV-Indexwerten, beispielsweise aus öffentlich verfügbaren Daten oder aus einem in dem Gerät 10 eingebauten (nicht gezeigten) UV-Sensor.

Der Prozessor verarbeitet alle Messwerte, weiteren Messwerte, persönlichen Daten und externen Daten und berechnet ein Ergebnis, das für die Empfehlung eines idealen Wertes für den Sonnenschutzfaktor verwendet wird. Dieser Wert kann von dem Gerät selbst oder von der externen Datenverarbeitungsvorrichtung 100 angezeigt werden.

Der Prozessor kann ferner anhand der Messwerte, der weiteren Messwerte, der persönlichen Daten der Person, der weiteren externen Daten, Information über den Hauttyp und/oder das Hautprofil der Person P generieren und auf dieser Basis die Ausbringungsmenge der kosmetischen Substanz S entsprechend variieren.

Damit besteht nicht nur die Möglichkeit die individuell optimale Ausbringungsmenge für die kosmetische Substanz S zu ermitteln und zu realisieren. Es besteht vielmehr die Möglichkeit der Person P, welche das Gerät 10 verwenden will, individuelle Empfehlungen in Bezug auf die Auswahl der kosmetischen Substanz S zu geben. Es können Zeitfenster vorgegeben werden, in denen idealerweise ein Auftrag der Substanz S erfolgen sollte. Es können Erinnerungssignale erzeugt werden, um die Person P an einen erneuten Auftrag der Substanz S zu erinnern.

In einer weiteren Ausgestaltung des erfindungsgemäßen Verfahrens kann das Gerät 10 eine selbstlernende Diagnose durchführen und die Ausbringungsmenge der kosmetischen Substanz S stets optimal auf die das Gerät 10 jeweils verwendende Person P anpassen. Es handelt sich mithin um ein iterativ lernendes Verfahren.

Eine andere Variante sieht vor, dass der Prozessor 70 während der Verwendung des Gerätes 10 anhand der Information über den Hauttyp und/oder das Hautprofil der Person P, der persönlichen Informationen, des geeigneten Sonnenschutzfaktors, der Information über die geeignete kosmetische Substanz S und/oder die Zeit-Information die Ausbringungsmenge der zu versprühenden kosmetischen Substanz aus dem Gerät anpasst oder variiert.

Das Gerät 10 kann damit auf Basis der verfügbaren Messwerte, Informationen und Daten die Person P zur richtigen Zeit daran erinnern, dass sie sich eincremen soll, wann sie sich eine Sonnenbrille aufsetzen soll, wann sie komplett aus der Sonne gehen soll, u.dgl.

In einer weiteren Abwandlung kann das Gerät 10 auch daran erinnern, wenn die Person P erneut die kosmetische Substanz auftragen soll, beispielsweise, weil der Sonnenschutz sich typischerweise abträgt durch Bewegung und Baden. Der genaue Zeitpunkt des Sonnenschutz-Alarms wird dabei bestimmt durch die Messwerte, Informationen und Daten, die dem Gerät 10 bzw. dem Prozessor 70 übermittelt worden sind. So können die persönlichen Informationen auch Daten über ein Bewegungsprofil (bei sportlicher Betätigung) oder weitere persönliche Daten umfassen (z.B. Schwitzverhalten).

Auf diese Weise sorgt das Gerät 10 mit dem erfindungsgemäßen Verfahren dafür, dass ein Sonnenbrand wirksam vermieden wird. Das Gerät sorgt dafür, dass die Person stets den richtigen Sonnenschutzfaktor verwendet und vor allem die richtige Menge an kosmetischer Substanz S im richtigen Zeitpunkt aufträgt. Über die Bewegungsprofile kann zudem erkannt werden, ob ggf. die eine oder andere Hautstelle nicht ausreichend oder gar nicht eingecremt worden ist.

Um die Anwendung und den Auftrag der kosmetischen Substanz S weiter zu individualisieren, zu personalisieren und/oder zu optimieren, sieht das erfindungsgemäße Verfahren vor, dass die Person P nach der Verwendung des Geräts 10 Antworten auf Fragen in die externe Datenverarbeitungsvorrichtung 100 eingeben muss.

Die Antworten der Person werden von der externen Datenverarbeitungsvorrichtung 100 analysiert und/oder ausgewertet und zu weiteren persönlichen Informationen verarbeitet, wobei die weiteren persönlichen Informationen von der externen Datenverarbeitungsvorrichtung 100 über die Sende- und Empfangseinheit 90 des Gerätes 10 an den Prozessor übermittelt und zu den persönlichen Daten der Person hinzugefügt werden.

In einer noch anderen Ausführungsform des erfindungsgemäßen Verfahrens legt die externe Datenverarbeitungsvorrichtung die Messwerte, die weiteren Messwerte, die persönlichen Daten der Person, die weiteren externen Daten, das wenigstens eine von dem Prozessor berechnete Ergebnis und/oder das Bewegungsprofil des Gerätes in einer Datenbank ab. Diese kann in einer Cloud gespeichert sein.

Aus diesen Daten kann das Verfahren erkennen, dass z.B. viele vergleichbare Personen (z.B. mit gleichem Hauttyp, am gleichen Ort, bei vergleichbarer UV-Belastung) sich nicht ausreichend eingecremt haben. Diese Information wird dann lernend an die gesamte Personengruppe weitergegeben und das jeweilige Gerät wird entsprechend der individuellen Person verwendet, d.h. es wird - unter Berücksichtigung der Messwerte, der weiteren Messwerte, der persönlichen Daten, der weiteren externen Daten und/oder der Referenzwerte - die für die jeweilige Person optimale Ausbringungsmenge an kosmetischer Substanz generiert.

Über die Erfassung dieser Nutzungsdaten aller Geräte 10 lassen sich Nutzergruppen identifizieren und aus dem Verhalten ähnlicher Nutzergruppen Empfehlungen für den individuellen Nutzer ableiten.

Die Erfindung ist nicht auf eine der vorbeschriebenen Ausführungsformen beschränkt, sondern in vielfältiger Weise abwandelbar, solange die daraus resultierenden Ausführungsformen unter dem Wortlaut der beigefügten Ansprüche fallen.

So können auch bei der Erfassung der Beschleunigung und Neigung des Gerätes 10 zwei oder drei Sensoren verwendet werden.

Bevorzugt wird für jede Art von Messwert ein separater Sensor vorgesehen, wobei für einen Messwert auch mehrere Sensoren eingesetzt werden können, deren Messwerte sich zu einer Information ergänzen.

In einer Weiterbildung des erfindungsgemäßen Verfahrens kann der wenigstens eine Sensor die Spannung an der Hochspannungsquelle des Gerätes erfassen und diese Information an den Prozessor weiterleiten.

Denkbar ist auch der Einsatz von optischen oder akustischen Signalgebern direkt im Gerät, die eine zuverwendende kosmetische Substanz anzeigen, an einen Auftrag der Substanz erinnern oder sonstige Informationen an die das Gerät nutzende Person geben..

Man erkennt, dass die Erfindung ein Verfahren zum Auftragen einer kosmetischen Substanz S auf die Haut H einer Person P betrifft, unter Verwendung eines Gerätes 10 zum elektrostatischen Versprühen der kosmetischen Substanz S. Das Gerät 10 hat einen Behälter 20, eine Düsenanordnung 30, eine Fördervorrichtung 40, eine Hochspannungsquelle 50, einen Prozessor 70 zum Verarbeiten von Messwerten, Daten und/oder Referenzwerten, einen Speicher 80 sowie eine Sende- und Empfangseinheit 90 zum Übertragen der Messwerte, Daten und/oder Referenzwerte an eine externe Datenverarbeitungsvorrichtung 100 und/oder zum Empfangen von weiteren Messwerten, Daten und/oder Referenzwerten von der externen Datenverarbeitungsvorrichtung 100. Das Verfahren sieht vor, dass die Messwerte und ggf. weitere Messwerte, persönliche Daten der Person P, weitere externe Daten und/oder Referenzwerte erfasst und/oder ermittelt und an den Prozessor 70 übermittelt werden, wobei der Prozessor 70 die Messwerte, die weiteren Messwerte, die persönlichen Daten, die weiteren externen Daten und/oder die Referenzwerte zu wenigstens einem Ergebnis verarbeitet und anhand dieser Ergebnisse die Ausbringungsmenge der zu versprühenden kosmetischen Substanz S aus dem Gerät 10 variiert.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

## Patentansprüche

1. Nicht-therapeutisches Verfahren zum Auftragen einer kosmetischen Substanz (S) auf die Haut (H) einer Person (P), unter Verwendung eines Gerätes (10) zum elektrostatischen Versprühen der kosmetischen Substanz (S),
das Gerät (10) aufweisend,
▪ einen Behälter (20) für die kosmetische Substanz (S),
▪ eine Düsenanordnung (30) zum Versprühen der kosmetischen Substanz (S),
▪ eine Fördervorrichtung (40), zum Fördern der kosmetischen Substanz (S) von dem Behälter (20) zu der Düsenanordnung (30),
▪ eine Hochspannungsquelle (50),
▪ einen Prozessor (70) zum Verarbeiten von Messwerten, Daten und/oder Referenzwerten,
▪ einen Speicher (80) zum Speichern der Messwerte, Daten und/oder Referenzwerte,
▪ eine Sende- und Empfangseinheit (90) zum Übertragen der Messwerte, Daten und/oder Referenzwerte an eine externe Datenverarbeitungsvorrichtung (100) und/oder zum Empfangen von weiteren Messwerten, Daten und/oder Referenzwerten von der externen Datenverarbeitungsvorrichtung (100),
das Verfahren aufweisend folgende Schritte,
a) die Messwerte, weitere Messwerte, persönliche Daten der Person (P), weitere externe Daten und/oder Referenzwerte werden erfasst und/oder ermittelt und an den Prozessor (70) übermittelt,
b) der Prozessor (70) verarbeitet die Messwerte, die weiteren Messwerte, die persönlichen Daten, die weiteren externen Daten und/oder die Referenzwerte zu wenigstens einem Ergebnis,
c) aus den Messwerten, den weiteren Messwerten, den persönlichen Daten der Person, den weiteren externen Daten und/oder den von dem Prozessor berechneten Ergebnissen wird eine Information über eine für die Person geeignete kosmetische Substanz generiert,
d) wobei über die externe Datenverarbeitungsvorrichtung oder über das Gerät selbst Informationen an die Person abgegeben werden, die das Gerät verwendet oder verwenden will, wobei sich diese Informationen auf die kosmetische Substanz beziehen,
e) der Prozessor (70) variiert anhand der Ergebnisse die Ausbringungsmenge der zu versprühenden kosmetischen Substanz (S) aus dem Gerät (10).

2. Nicht-therapeutisches Verfahren nach Anspruch 1, **dadurch gekennzeichnet, dass** die geeignete kosmetische Substanz von dem Prozessor und/oder von der externen Datenverarbeitungsvorrichtung bestimmt wird

3. Nicht-therapeutisches Verfahren nach Anspruch 1 oder 2, **dadurch gekennzeichnet, dass** die Information über die geeignete kosmetische Substanz von der externen Datenverarbeitungsvorrichtung über die Sende- und Empfangseinheit des Gerätes an den Prozessor übermittelt und zu den persönlichen Daten der Person und/oder zu den weiteren externen Daten hinzugefügt wird,

4. Nicht-therapeutisches Verfahren nach einem der vorherigen Ansprüche, **dadurch gekennzeichnet, dass** der Prozessor (70) die Ausbringungsmenge der zu versprühenden kosmetischen Substanz (S) durch Regelung der Fördervorrichtung (40) variiert.

5. Nicht-therapeutisches Verfahren nach einem der vorherigen Ansprüche, **dadurch gekennzeichnet, dass** der Prozessor (70) die Ausbringungsmenge der zu versprühenden kosmetischen Substanz (S) durch Regelung der Hochspannungsquelle (50) variiert.

6. Nicht-therapeutisches Verfahren nach einem der vorherigen Ansprüche, **dadurch gekennzeichnet, dass** der Prozessor (70) die Ausbringungsmenge der zu versprühenden kosmetischen Substanz (S) durch Beeinflussung der Düsenanordnung (30) variiert.

7. Nicht-therapeutisches Verfahren nach einem der vorherigen Ansprüche, **dadurch gekennzeichnet, dass** wenigstens ein weiterer Sensor (66) die weiteren Messwerte erfasst, wobei der Prozessor (70) bei der Berechnung des wenigstens einen Ergebnisses die weiteren Messwerte berücksichtigt.

8. Nicht-therapeutisches Verfahren nach einem der vorherigen Ansprüche, **dadurch gekennzeichnet, dass** der Prozessor (70) bei der Berechnung des wenigstens einen Ergebnisses die persönlichen Daten der Person (P) berücksichtigt.

9. Nicht-therapeutisches Verfahren nach Anspruch 8, **dadurch gekennzeichnet, dass** die persönlichen Daten der Person (P) in die externe Datenverarbeitungsvorrichtung (100) eingegeben und/oder von dieser anhand einer Datenbank, im Internet, einer sonstigen Datenquelle, o.dgl. ermittelt werden.

10. Nicht-therapeutisches Verfahren nach einem der vorherigen Ansprüche, **dadurch gekennzeichnet, dass** der Prozessor (70) bei der Berechnung des wenigstens einen Ergebnisses die weiteren externen Daten berücksichtigt.

11. Nicht-therapeutisches Verfahren nach Anspruch 10, **dadurch gekennzeichnet, dass** die weiteren externen Daten in die externe Datenverarbeitungsvorrichtung (100) eingegeben oder von dieser anhand einer Datenbank, im Internet, einer sonstigen Datenquelle, o.dgl. ermittelt werden.

12. Nicht-therapeutisches Verfahren nach einem der vorherigen Ansprüche, **dadurch gekennzeichnet, dass** der Prozessor (70) bei der Berechnung des wenigstens einen Ergebnisses die Messwerte, die weiteren Messwerte, die persönlichen Daten und/oder die weiteren externen Daten mit Referenzwerten vergleicht.

13. Nicht-therapeutisches Verfahren nach einem der vorherigen Ansprüche, **dadurch gekennzeichnet, dass** aus den Messwerten, den weiteren Messwerten, den persönlichen Daten der Person (P), der weiteren externen Daten, den von dem Prozessor berechneten Ergebnissen und/oder dem Bewegungsprofil des Gerätes (10) eine Information über den Hauttyp und/oder das Hautprofil der Person (P) generiert wird.

14. Nicht-therapeutisches Verfahren nach einem der vorherigen Ansprüche, **dadurch gekennzeichnet, dass** aus den Messwerten, den weiteren Messwerten, den persönlichen Daten der Person (P), der weiteren externen Daten, den von dem Prozessor berechneten Ergebnissen und/oder dem Bewegungsprofil des Gerätes (10) eine Information über einen für die Person (P) geeigneten Sonnenschutzfaktor generiert wird.

15. Nicht-therapeutisches Verfahren nach einem der vorherigen Ansprüche, **dadurch gekennzeichnet, dass** aus den Messwerten, den weiteren Messwerten, den persönlichen Daten der Person (P), der weiteren externen Daten, den von dem Prozessor berechneten Ergebnissen und/oder dem Bewegungsprofil des Gerätes (10) eine Information über eine für die Person (P) geeignete kosmetische Substanz (S) generiert wird.

16. Nicht-therapeutisches Verfahren nach einem der vorherigen Ansprüche, **dadurch gekennzeichnet, dass** aus den Messwerten, den weiteren Messwerten, den persönlichen Daten der Person (P), der weiteren externen Daten, den von dem Prozessor berechneten Ergebnissen und/oder dem Bewegungsprofil des Gerätes (10) eine Zeit-Information für die Person (P) generiert wird.

17. Nicht-therapeutisches Verfahren nach einem der vorherigen Ansprüche, **dadurch gekennzeichnet, dass** die Information über den Hauttyp und/oder das Hautprofil der Person (P), die persönlichen Informationen, der geeignete Sonnenschutzfaktor, die geeignete kosmetische Substanz (S) und/oder die Zeit-Information von der externen Datenverarbeitungsvorrichtung (100) über die Sende- und Empfangseinheit (90) des Gerätes (10) an den Prozessor (70) übermittelt und zu den persönlichen Daten der Person (P) und/oder zu den weiteren externen Daten hinzugefügt werden.

18. Gerät (10) zum Auftragen einer kosmetischen Substanz (S) auf die Haut (H) einer Person (P), aufweisend
a) einen Behälter (20) für die kosmetische Substanz (S),
b) eine Düsenanordnung (30) zum Versprühen der kosmetischen Substanz (S),
c) eine Fördervorrichtung (40), zum Fördern der kosmetischen Substanz (S) von dem Behälter zu der Düsenanordnung (30),
d) eine Hochspannungsquelle (50),
e) einen Prozessor (70) zum Verarbeiten von Messwerten, wobei das Gerät (10) wenigstens einen Sensor (60) aufweist und wobei die Messwerte von dem wenigstens einen Sensor (60) während des Auftragens der kosmetischen Substanz (S) auf die Haut (H) der Person (P) erfasst werden,
f) einen Speicher (80) zum Speichern der Messwerte,
g) eine Sende- und Empfangseinheit (90) zum Übertragen der Messwerte an eine externe Datenverarbeitungsvorrichtung (100) und/oder zum Empfangen von weiteren Messwerten, weiteren persönlichen Daten der Person (P) und/oder weiteren externen Daten von der externen Datenverarbeitungsvorrichtung (100),
h) wobei die Sende- und Empfangseinheit (90) eingerichtet und ausgebildet ist, die Messwerte, weitere Messwerte, weitere persönliche Daten der Person (P) und/oder weitere externe Daten zu erfassen und/oder zu ermitteln und an den Prozessor (70) zu übermitteln,
i) wobei der Prozessor (70) eingerichtet und ausgebildet ist, die Messwerte, die weiteren Messwerte, die weiteren persönlichen Daten der Person (P) und/oder die weiteren externen Daten zu wenigstens einem Ergebnis zu verarbeiten,
j) wobei der Prozessor (70) zum Verarbeiten von Referenzwerten eingerichtet ist,
k) wobei der Prozessor (70) dazu eingerichtet ist, die Messwerte, die Referenzwerte, die weiteren Messwerte, die weiteren persönlichen Daten, die weiteren externen Daten und/oder die weiteren Referenzwerte zu wenigstens einem Ergebnis zu verarbeiten,
l) wobei der Prozessor (70) bei der Berechnung des wenigstens einen Ergebnisses die Messwerte, die weiteren Messwerte, die persönlichen Daten und/oder die weiteren externen Daten mit den Referenzwerten vergleicht,
m) wobei der Prozessor (70) weiter dazu eingerichtet und ausgebildet ist, anhand des wenigstens einen Ergebnisses die Ausbringungsmenge der zu versprühenden kosmetischen Substanz (S) aus dem Gerät (10) zu variieren, und
n) wobei das Gerät (10) dazu ausgebildet und eingerichtet ist Informationen an die Person (P), die das Gerät verwendet oder verwenden will, über die externe Datenverarbeitungsvorrichtung (100) oder über das Gerät (10) selbst abzugeben.

19. Vorrichtung zum Auftragen einer kosmetischen Substanz (S) auf die Haut (H) einer Person (P), insbesondere durch ein Verfahren nach einem der Ansprüche 1 bis 17, umfassend ein Gerät (10) nach Anspruch 18 sowie eine externe Datenverarbeitungsvorrichtung (100) wie bspw. ein Computer, ein Tablet, ein Smartphone oder dgl., wobei die externe Datenverarbeitungsvorrichtung dazu ausgebildet und eingerichtet ist Informationen an die Person (P), die das Gerät verwendet oder verwenden will, abzugeben.

## Claims

1. A non-therapeutic method for applying a cosmetic substance (S) to the skin (H) of a person (P), using a device (10) for the electrostatic spraying of the cosmetic substance (S),
the device (10) comprising,
▪ a container (20) for the cosmetic substance (S),
▪ a nozzle assembly (30) for atomising the cosmetic substance (S),
▪ a delivery mechanism (40) for delivering the cosmetic substance (S) from the container (20) to the nozzle assembly (30),
▪ a high-voltage source (50),
▪ a processor (70) for processing measured values, data and/or reference values,
▪ a memory (80) for storing the measured values, data and/or reference values,
▪ a transmission and reception unit (90) for transmitting the measured values, data and/or reference values to an external data processing device (100) and/or for receiving further measured values, data and/or reference values from the external data processing device (100),
the method comprising the following steps:
a) the measured values, further measured values, personal data of the person (P), further external data and/or reference values are recorded and/or determined and transmitted to the processor (70),
b) the processor (70) processes the measured values, the further measured values, the personal data, the further external data and/or the reference values to produce at least one result,
c) information regarding a cosmetic substance suitable for the person is generated from the measured values, the further measured values, the personal data of the person, the further external data and/or the results calculated by the processor,
d) whereby information relating to the cosmetic substance is provided, via the external data-processing device or via the device itself, to the person who is using or intends to use the device,
e) the processor (70) varies the dispensing quantity of the cosmetic substance (S) to be sprayed from the device (10) on the basis of the results.

2. Non-therapeutic method according to claim 1, **characterised in that** the suitable cosmetic substance is determined by the processor and/or by the external data processing device

3. A non-therapeutic method according to claim 1 or 2, **characterised in that** the information regarding the suitable cosmetic substance is transmitted from the external data processing device to the processor via the device's transmission and reception unit and is added to the person's personal data and/or to the further external data,

4. A non-therapeutic method according to one of the preceding claims, **characterised in that** the processor (70) varies the dispensing quantity of the cosmetic substance (S) to be sprayed by controlling the delivery device (40).

5. A non-therapeutic method according to any of the preceding claims, **characterised in that** the processor (70) varies the discharge rate of the cosmetic substance (S) to be sprayed by controlling the high-voltage source (50).

6. A non-therapeutic method according to any one of the preceding claims, **characterised in that** the processor (70) varies the discharge rate of the cosmetic substance (S) to be sprayed by influencing the nozzle arrangement (30).

7. A non-therapeutic method according to one of the preceding claims, **characterised in that** at least one further sensor (66) records the further measured values, wherein the processor (70) takes the further measured values into account when calculating the at least one result.

8. V A non-therapeutic method according to one of the preceding claims, **characterised in that** the processor (70) takes into account the personal data of the person (P) when calculating the at least one result.

9. A non-therapeutic method according to claim 8, **characterised in that** the personal data of the person (P) is entered into the external data processing device (100) and/or is determined by the latter on the basis of a database, the Internet, another data source, or the like.

10. A non-therapeutic method according to any of the preceding claims, **characterised in that** the processor (70) takes the further external data into account when calculating the at least one result.

11. A non-therapeutic method according to claim 10, **characterised in that** the further external data are entered into the external data processing device (100) or are determined by it on the basis of a database, the Internet, another data source, or the like.

12. A non-therapeutic method according to any of the preceding claims, **characterised in that**, when calculating the at least one result, the processor (70) compares the measured values, the further measured values, the personal data and/or the further external data with reference values.

13. A non-therapeutic method according to any one of the preceding claims, **characterised in that** information regarding the skin type and/or skin profile of the person (P) is generated from the measured values, the further measured values, the personal data of the person (P), the further external data, the results calculated by the processor and/or the movement profile of the device (10).

14. A non-therapeutic method according to any of the preceding claims, **characterised in that** information regarding a sun protection factor suitable for the person (P) is generated from the measured values, the further measured values, the personal data of the person (P), the further external data, the results calculated by the processor and/or the movement profile of the device (10).

15. A non-therapeutic method according to any of the preceding claims, **characterised in that** information regarding a cosmetic substance (S) suitable for the person (P) is generated from the measured values, the further measured values, the personal data of the person (P), the further external data, the results calculated by the processor and/or the movement profile of the device (10).

16. A non-therapeutic method according to any of the preceding claims, **characterised in that** time-related information for the person (P) is generated from the measured values, the further measured values, the personal data of the person (P), the further external data, the results calculated by the processor and/or the movement profile of the device (10).

17. A non-therapeutic method according to any one of the preceding claims, **characterised in that** the information regarding the skin type and/or skin profile of the person (P), the personal information, the suitable sun protection factor, the suitable cosmetic substance (S) and/or the time information is transmitted from the external data processing device (100) via the transmission and reception unit (90) of the device (10) to the processor (70) and added to the personal data of the person (P) and/or to the other external data.

18. A device (10) for applying a cosmetic substance (S) to the skin (H) of a person (P), comprising
a) a container (20) for the cosmetic substance (S),
b) a nozzle assembly (30) for spraying the cosmetic substance (S),
c) a delivery device (40) for delivering the cosmetic substance (S) from the container to the nozzle assembly (30),
d) a high-voltage source (50),
e) a processor (70) for processing measured values, wherein the device (10) comprises at least one sensor (60) and wherein the measured values are recorded by the at least one sensor (60) whilst the cosmetic substance (S) is being applied to the skin (H) of the person (P),
f) a memory (80) for storing the measured values,
g) a transmission and reception unit (90) for transmitting the measured values to an external data-processing device (100) and/or for receiving further measured values, further personal data relating to the person (P) and/or further external data from the external data-processing device (100),
h) wherein the transmission and reception unit (90) is configured and designed to acquire and/or determine the measured values, further measured values, further personal data of the person (P) and/or further external data, and to transmit them to the processor (70),
i) wherein the processor (70) is configured and designed to process the measured values, the further measured values, the further personal data of the person (P) and/or the further external data to produce at least one result,
j) wherein the processor (70) is configured to process reference values,
k) wherein the processor (70) is configured to process the measured values, the reference values, the further measured values, the further personal data, the further external data and/or the further reference values to produce at least one result,
l) wherein, when calculating the at least one result, the processor (70) compares the measured values, the further measured values, the personal data and/or the further external data with the reference values,
m) wherein the processor (70) is further configured and designed to vary, on the basis of the at least one result, the discharge rate of the cosmetic substance (S) to be sprayed from the device (10), and
n) wherein the device (10) is designed and configured to provide information to the person (P) who is using or intends to use the device, either via the external data-processing device (100) or via the device (10) itself.

19. A device for applying a cosmetic substance (S) to the skin (H) of a person (P), in particular by a method according to any one of claims 1 to 17, comprising a device (10) according to claim 18 and an external data-processing device (100) such as, for example, a computer, a tablet, a smartphone or the like, wherein the external data-processing device is designed and configured to provide information to the person (P) who is using or intends to use the device.

## Revendications

1. Procédé non thérapeutique d'application d'une substance cosmétique (S) sur la peau (H) d'une personne (P), utilisant un appareil (10) destiné à la pulvérisation électrostatique de la substance cosmétique (S),
ledit appareil (10) comprenant :
▪ un réservoir (20) destiné à contenir la substance cosmétique (S),
▪ un ensemble de buses (30) destiné à pulvériser la substance cosmétique (S),
▪ un dispositif d'alimentation (40) destiné à acheminer la substance cosmétique (S) du réservoir (20) vers l'ensemble de buses (30),
▪ une source de haute tension (50),
▪ un processeur (70) destiné à traiter des valeurs de mesure, des données et/ou des valeurs de référence,
▪ une mémoire (80) destinée à stocker les valeurs de mesure, les données et/ou les valeurs de référence,
▪ une unité d'émission et de réception (90) destinée à transmettre les valeurs de mesure, les données et/ou les valeurs de référence à un dispositif externe de traitement de données (100) et/ou à recevoir d'autres valeurs de mesure, données et/ou valeurs de référence provenant du dispositif externe de traitement de données (100),
le procédé comprenant les étapes suivantes :
a) les valeurs mesurées, d'autres valeurs mesurées, les données personnelles de la personne (P), d'autres données externes et/ou des valeurs de référence sont enregistrées et/ou déterminées, puis transmises au processeur (70),
b) le processeur (70) traite les valeurs de mesure, les autres valeurs de mesure, les données personnelles, les autres données externes et/ou les valeurs de référence pour obtenir au moins un résultat,
c) à partir des valeurs de mesure, des autres valeurs de mesure, des données personnelles de la personne, des autres données externes et/ou des résultats calculés par le processeur, une information concernant une substance cosmétique adaptée à la personne est générée,
d) des informations relatives à la substance cosmétique étant transmises, via le dispositif externe de traitement de données ou via l'appareil lui-même, à la personne qui utilise ou souhaite utiliser l'appareil, ces informations portant sur la substance cosmétique,
e) le processeur (70) fait varier, en fonction des résultats, la quantité de substance cosmétique (S) à pulvériser à partir de l'appareil (10).

2. Procédé non thérapeutique selon la revendication 1, **caractérisé en ce que** la substance cosmétique adaptée est déterminée par le processeur et/ou par le dispositif externe de traitement de données

3. Procédé non thérapeutique selon la revendication 1 ou 2, **caractérisé en ce que** les informations relatives à la substance cosmétique appropriée sont transmises au processeur par le dispositif externe de traitement de données via l'unité d'émission et de réception de l'appareil, puis ajoutées aux données personnelles de la personne et/ou aux autres données externes,

4. Procédé non thérapeutique selon l'une des revendications précédentes, **caractérisé en ce que** le processeur (70) fait varier le débit de la substance cosmétique (S) à pulvériser en régulant le dispositif d'alimentation (40).

5. Procédé non thérapeutique selon l'une des revendications précédentes, **caractérisé en ce que** le processeur (70) fait varier le débit de la substance cosmétique (S) à pulvériser en régulant la source haute tension (50).

6. Procédé non thérapeutique selon l'une des revendications précédentes, **caractérisé en ce que** le processeur (70) fait varier le débit de la substance cosmétique (S) à pulvériser en agissant sur le dispositif de buses (30).

7. Procédé non thérapeutique selon l'une des revendications précédentes, **caractérisé en ce qu'**au moins un capteur supplémentaire (66) détecte les autres valeurs de mesure, le processeur (70) prenant en compte lesdites autres valeurs de mesure lors du calcul dudit au moins un résultat.

8. V Procédé non thérapeutique selon l'une des revendications précédentes, **caractérisé en ce que** le processeur (70) prend en compte les données personnelles de la personne (P) lors du calcul dudit au moins un résultat.

9. Procédé non thérapeutique selon la revendication 8, **caractérisé en ce que** les données personnelles de la personne (P) sont saisies dans le dispositif externe de traitement de données (100) et/ou sont déterminées par celui-ci à l'aide d'une base de données, sur Internet, d'une autre source de données, ou similaire.

10. Procédé non thérapeutique selon l'une des revendications précédentes, **caractérisé en ce que** le processeur (70) prend en compte les autres données externes lors du calcul dudit au moins un résultat.

11. Procédé non thérapeutique selon la revendication 10, **caractérisé en ce que** les données externes supplémentaires sont saisies dans le dispositif de traitement de données externe (100) ou sont déterminées par celui-ci à partir d'une base de données, d'Internet, d'une autre source de données ou similaire.

12. Procédé non thérapeutique selon l'une des revendications précédentes, **caractérisé en ce que**, lors du calcul dudit au moins un résultat, le processeur (70) compare les valeurs mesurées, les autres valeurs mesurées, les données personnelles et/ou les autres données externes à des valeurs de référence.

13. Procédé non thérapeutique selon l'une des revendications précédentes, **caractérisé en ce qu'**une information sur le type de peau et/ou le profil cutané de la personne (P) est générée à partir des valeurs de mesure, des autres valeurs de mesure, des données personnelles de la personne (P), des autres données externes, des résultats calculés par le processeur et/ou du profil de mouvement de l'appareil (10).

14. Procédé non thérapeutique selon l'une des revendications précédentes, **caractérisé en ce qu'**une information concernant un indice de protection solaire adapté à la personne (P) est générée à partir des valeurs de mesure, des autres valeurs de mesure, des données personnelles de la personne (P), des autres données externes, des résultats calculés par le processeur et/ou du profil de mouvement de l'appareil (10).

15. Procédé non thérapeutique selon l'une des revendications précédentes, **caractérisé en ce qu'**une information concernant une substance cosmétique (S) adaptée à la personne (P) est générée à partir des valeurs de mesure, des autres valeurs de mesure, des données personnelles de la personne (P), des autres données externes, des résultats calculés par le processeur et/ou du profil de mouvement de l'appareil (10).

16. Procédé non thérapeutique selon l'une des revendications précédentes, **caractérisé en ce qu'**une information temporelle destinée à la personne (P) est générée à partir des valeurs mesurées, des autres valeurs mesurées, des données personnelles de la personne (P), des autres données externes, des résultats calculés par le processeur et/ou du profil de mouvement de l'appareil (10).

17. Procédé non thérapeutique selon l'une des revendications précédentes, **caractérisé en ce que** les informations relatives au type de peau et/ou au profil cutané de la personne (P), les informations personnelles, l'indice de protection solaire adapté, la substance cosmétique (S) adaptée et/ou l'information temporelle sont transmises par le dispositif externe de traitement des données (100) via l'unité d'émission et de réception (90) de l'appareil (10) vers le processeur (70) et sont ajoutées aux données personnelles de la personne (P) et/ou aux autres données externes.

18. Appareil (10) destiné à appliquer une substance cosmétique (S) sur la peau (H) d'une personne (P), comprenant
a) un réservoir (20) destiné à contenir la substance cosmétique (S),
b) un ensemble de buses (30) destiné à pulvériser la substance cosmétique (S),
c) un dispositif d'alimentation (40) destiné à acheminer la substance cosmétique (S) du réservoir vers le dispositif de pulvérisation (30),
d) une source de haute tension (50),
e) un processeur (70) destiné à traiter des valeurs de mesure, l'appareil (10) comportant au moins un capteur (60) et les valeurs de mesure étant enregistrées par ledit au moins un capteur (60) pendant l'application de la substance cosmétique (S) sur la peau (H) de la personne (P),
f) une mémoire (80) destinée à stocker les valeurs mesurées,
g) une unité d'émission et de réception (90) destinée à transmettre les valeurs mesurées à un dispositif externe de traitement de données (100) et/ou à recevoir d'autres valeurs mesurées, d'autres données personnelles de la personne (P) et/ou d'autres données externes provenant du dispositif externe de traitement de données (100),
h) l'unité d'émission et de réception (90) étant agencée et conçue pour acquérir et/ou déterminer les valeurs mesurées, d'autres valeurs mesurées, d'autres données personnelles de la personne (P) et/ou d'autres données externes, et pour les transmettre au processeur (70),
i) le processeur (70) étant configuré et conçu pour traiter les valeurs de mesure, les autres valeurs de mesure, les autres données personnelles de la personne (P) et/ou les autres données externes afin d'obtenir au moins un résultat,
j) le processeur (70) étant configuré pour traiter des valeurs de référence,
k) le processeur (70) étant conçu pour traiter les valeurs mesurées, les valeurs de référence, les autres valeurs mesurées, les autres données personnelles, les autres données externes et/ou les autres valeurs de référence afin d'obtenir au moins un résultat,
l) le processeur (70) comparant, lors du calcul dudit au moins un résultat, les valeurs de mesure, les autres valeurs de mesure, les données personnelles et/ou les autres données externes aux valeurs de référence,
m) le processeur (70) étant en outre conçu et configuré pour faire varier, sur la base dudit au moins un résultat, le débit de la substance cosmétique (S) à pulvériser à partir de l'appareil (10), et
n) l'appareil (10) étant conçu et agencé pour fournir des informations à la personne (P) qui utilise ou souhaite utiliser l'appareil, soit par l'intermédiaire du dispositif externe de traitement de données (100), soit par l'intermédiaire de l'appareil (10) lui-même.

19. Dispositif destiné à appliquer une substance cosmétique (S) sur la peau (H) d'une personne (P), notamment selon un procédé visé par l'une des revendications 1 à 17, comprenant un appareil (10) selon la revendication 18 ainsi qu'un dispositif externe de traitement de données (100) tel qu'un ordinateur, une tablette, un smartphone ou similaire, le dispositif externe de traitement de données étant conçu et configuré pour fournir des informations à la personne (P) qui utilise ou souhaite utiliser l'appareil.
